# EUROPÄISCHE PATENTSCHRIFT

(11) **EP 2 507 205 B1**
(45) Veröffentlichungstag und Bekanntmachung des Hinweises auf die Patenterteilung: **16.07.2014**
(21) Anmeldenummer: 10787380.4
(22) Anmeldetag: 30.11.2010
(51) Int. Cl.: C07C 263/04, C07C 265/14

(54) **VERFAHREN ZUR HERSTELLUNG VON ISOCYANATEN DURCH THERMISCHE SPALTUNG VON CARBAMATEN**
METHOD FOR PRODUCING ISOCYANATES BY THERMALLY SPLITTING CARBAMATES
PROCÉDÉ DE FABRICATION D'ISOCYANATES PAR DISSOCIATION THERMIQUE DE CARBAMATES

(30) Priorität: 01.12.2009 EP 09177643
(43) Veröffentlichungstag der Anmeldung: 10.10.2012
(73) Patentinhaber: BASF SE, 67056 Ludwigshafen (DE)
(72) Erfinder: BOCK, Michael, 67152 Ruppertsberg (DE); STROEFER, Eckhard, 68163 Mannheim (DE); BAUMANN, Robert, 68529 Mannheim (DE); FRANZKE, Axel, 68161 Mannheim (DE); PFEFFINGER, Joachim, 67071 Ludwigshafen (DE)
(86) Internationale Anmeldenummer: PCT/EP2010/068500
(87) Internationale Veröffentlichungsnummer: WO 2011/067242

(56) Entgegenhaltungen:
- EP-A1- 0 078 005
- EP-A1- 0 795 543
- EP-A1- 1 403 248
- WO-A1-03/074477
- US-A- 3 743 941

## Beschreibung

Die Erfindung betrifft ein Verfahren zur Herstellung von Isocyanaten durch thermische Spaltung von Carbamaten, auch als Carbamidsäureester oder Urethane bekannt.

Die Carbamatspaltung gewinnt zunehmend an Bedeutung als phosgenfreies Verfahren zur Herstellung von Isocyanaten. Zur technischen Durchführung der Carbamatspaltung wurden unterschiedliche Apparate vorgeschlagen, insbesondere Kolonnen (in EP 0 795 543), Wirbelschichtreaktoren (in EP 555 628 und in DE 199 07 648), Fallfilm- oder Dünnschichtverdampfer (in EP 0 092 738). Die Carbamatspaltung kann in der Flüssig- oder in der Gasphase betrieben werden.

Problematisch bei der thermischen Spaltung von Carbamaten ist die Bildung von hochmolekularen Nebenkomponenten, die durch Weiterreaktion der Spaltprodukte entstehen. Diese können zu Ablagerungen in den Apparaten führen, damit den kontinuierlichen Betrieb einschränken und zu Ausbeuteverlusten führen. Die Rückstände enthalten insbesondere Allophanate und Isocyanurate.

Um diese Probleme zu vermeiden, müssen die Spaltprodukte Isocyanat und Alkohol aus dem Carbamat-Spaltgas möglichst schnell voneinander getrennt werden.

Es ist weiterhin bekannt, dass die Problematik der Rück- und Weiterreaktion in der Spaltung reduziert werden kann, indem die Carbamat-Spaltung in Gegenwart von Lösungsmitteln durchgeführt wird, da die Reaktionsgeschwindigkeit der Rückreaktion von Isocyanat und Alkohol (Urethanisierung) sowie auch der Weiterreaktionen bekanntermaßen vom Lösungsmittel und der Verdünnung durch das Lösungsmittel abhängig ist.

Beispielsweise finden sich in J.H. Saunders und K.C. Frisch: Polyurethanes, Chemistry and Technology, 1962, S. 146, Tabelle 10, Angaben über die Reaktivität von Isocyanaten mit Alkoholen in Gegenwart unterschiedlicher Lösungsmittel. Durch Verdünnen der Carbamatspaltprodukte mit einem inerten Lösungsmittel wird die Bildung hochmolekularer Folgeprodukte zurückgedrängt; gleichzeitig dient das Lösungsmittel zur Ausschleusung dieser Nebenkomponenten und das Apparate-Fouling wird reduziert.

Als Lösungsmittel sind insbesondere Hochsieder geeignet, d.h. Flüssigkeiten, deren Siedepunkte unter Verfahrensbedingungen mindestens 10°C, insbesondere mindestens 20°C oberhalb der Siedepunkte der Spaltprodukte der Carbamat-Spaltung liegen. Die EP-B 0 795 543 beschreibt besonders geeignete hochsiedende Lösungsmittel zur thermischen Spaltung von Carbamaten, die einen definierten Siedepunkt oder aber einen engen Siedebereich aufweisen, und die als Destillationsschnitt von thermostabilen Flüssigkeiten gewonnen werden, ausgewählt aus der Gruppe der ortho-, meta- und para-Isomeren von Phenoxydiphenyl. Durch Einsatz derartiger Lösungsmittel beim thermischen Spalten von Carbamaten in Kolonnen kann die Sumpftemperatur der Ko-Ionne, bei gleicher Spaltleistung und unveränderter mittlerer Temperatur im Reaktionsteil verringert werden, wodurch die Bildung von Neben- und Crackprodukten im Kolonnensumpf deutlich reduziert wird. Nachteilig an diesem Verfahren ist die Tatsache, dass Spaltung und Spaltgastrennung im gleichen Apparat durchgeführt werden, dass als Rücklauf am Kolonnenkopf der Alkohol verwendet wird und Phenoxybiphenyl kommerziell kaum verfügbar und damit teuer ist.

Die WO 03/074477 A1 beschreibt ein gattungsgemäßes Verfahren zur Herstellung von Isocyanaten durch thermische Spaltung von Carbamaten, wobei das erhaltene Carbamat-Spaltgas abgekühlt und zumindest teilweise kondensiert wird unter Einsatz eines Teilstromes des bei der Carbamatspaltung entstandenen Alkohols als flüssiges Kühl- und Kondensationsmittel.

Es ist auch bekannt, die Geschwindigkeit der Urethanbildung durch Zusatz von Inhibitoren zu reduzieren. Als Inhibitoren der Urethanbildung sind beispielsweise Salzsäure, Benzoylchlorid oder p-Toluolsulfonsäure bekannt (vgl. Örtel: Polyurethane, 2. Auflage, 3.4.2, S. 92).

Es war demgegenüber Aufgabe der Erfindung, ein verbessertes Verfahren zur thermischen Spaltung von Carbamaten in Gegenwart eines Zusatzstoffes zur Verfügung zu stellen, das zu einer verminderten Rückstandsmenge bei reduzierten spezifischen Energiekosten führt.

Die Aufgabe wird gelöst durch ein Verfahren zur Herstellung von Isocyanaten durch thermische Spaltung von Carbamaten, wobei ein Carbamat-Spaltgas, enthaltend das entsprechende Isocyanat und den entsprechenden Alkohol, erhalten wird, das dadurch gekennzeichnet ist, dass das Carbamat-Spaltgas in Gegenwart eines Ethers, der als Inhibitor für die Rückreaktion des Isocyanats mit dem Alkohol fungiert, gequencht wird.

Es wurde gefunden, dass es möglich ist, durch Einsatz von Ethern, die als Inhibitoren für die Rückreaktion des Isocyanats mit dem Alkohol, die bei der Carbamat-Spaltung gebildet werden, fungieren, die Geschwindigkeit der Rückreaktion zu reduzieren und damit die Probleme, die mit der Bildung von Nebenkomponenten verbunden sind, zu verringern. Gegenüber dem Einsatz von reinen Lösungsmitteln als Zusatzstoffen ist hierfür jedoch eine deutlich reduzierte spezifische Menge an Inhibitor erforderlich. Insbesondere ist dadurch auch die Abtrennung und Rückgewinnung des Zusatzstoffes gegenüber Zusatzstoffen, die lediglich als Lösungsmittel fungieren, wirtschaftlicher.

Erfindungsgemäß werden als Inhibitoren, die die Rückreaktion des Isocyanats mit dem Alkohol aus dem Carbamat-Spaltgas verlangsamen, Ether eingesetzt.

Es wird angenommen, dass Ether über die Ausbildung von Wasserstoffbrücken den Alkohol binden, so dass dieser für die Rückreaktion nicht oder nur in vermindertem Umfang zur Verfügung steht.

Bevorzugt wird als Ether ein Polyalkylenglykoldialkylether und/oder ein Polyalkylenglykoldiarylether eingesetzt.

Weiter bevorzugt ist der Polyalkylenglykoldialkylether ein Polyethylenglykoldialkylether. Besonders bevorzugt werden als als Polyethylenglykoldialkylether Diethylenglykoldimethylether, Triethylenglykoldimethylether oder Tetraethylenglykoldimethylether eingesetzt.

Besonders bevorzugt wird als Ether Tetraethylenglykoldimethylether eingesetzt.

Weiter bevorzugt können als Ether Polypropylenglykoldialkylether und/oder Polypropylenglykoldiarylether eingesetzt werden.

Als Polypropylenglykoldialkylether werden bevorzugt eine oder mehrere Substanzen, ausgewählt aus der nachfolgenden Aufzählung, eingesetzt: Dipropylenglykoldimethylether, Tripropylenglykoldimethylether oder Tetrapropylenglykoldimethylether.

In einer weiteren Ausführungsform werden cyclische oder aromatische Ether/Polyether eingesetzt, bevorzugt eine oder mehrere Substanzen, ausgewählt aus der nachfolgenden Aufzählung: Oxolan, Dioxolan, Trioxolan, Diarylether, Alkylarylether, Diphenylether, Ditolylether und Dibenzylether.

Bei den Carbamaten, die der Spaltung zugeführt werden, handelt es sich insbesondere um N,N'-Toluylen-bis(O-alkylurethan), N,N'-Toluylen-bis(O-alkoxalkylyurethan), N,N'-Toluylenbis(O-arylurethan) N,N'-1,5-Naphthalin-bis(O-dialkylurethan), N,N'-1,5-Naphthalin-bis(O-alkoxylalkylurethan), N,N'-1,5-Naphthalin-bis(O-arylurethan), Hexamethylen-bis(O-alkylurethan), Hexamethylen-bis(O-alkoxyalkylurethan), Isophoron-bis(O-alkylurethan), Isophoron-bis(O-alkoxyalkylurethan), 4,4'-Diphenylmethandi-O-alkylurethan oder 4,4'-Diphenylmethan-di-O-alkoxyalkylurethan.

Der als Zusatzstoff zur thermischen Spaltung eingesetzte Ether kann bevorzugt ein Schwersieder sein, d.h. eine Substanz, die einen Siedepunkt unter Betriebsbedingungen aufweist, der höher liegt als die Siedepunkte des Isocyanats und des Alkohols aus dem Carbamat-Spaltgas.

In einer weiteren Ausführungsform wird ein Ether eingesetzt, der ein Zwischensieder ist, d.h. der einen Siedepunkt unter Verfahrensbedingungen zwischen dem Siedepunkt des Isocyanats und dem Alkohol aufweist.

In einer weiteren Ausführungsform wird ein Ether als Zusatzstoff in der thermischen Spaltung eingesetzt, der einen Siedepunkt unterhalb der Siedepunkte des Alkohols und des Isocyanats aus dem Carbamat-Spaltgas aufweist, d.h. ein Leichtsieder.

Unter Quenchen wird, in bekannter Weise, das schnelle Abkühlen eines Gases mit einer Flüssigkeit bezeichnet. Vorliegend wird unter Quenchen insbesondere das Kühlen innerhalb von weniger als 10 Sekunden des Carbamat-Spaltgases mit einer großen Menge eines unter den Betriebsbedingungen der thermischen Spaltung flüssigen Ethers verstanden.

Nach der Kontaktphase des Carbamat-Spaltgases mit dem flüssigen Zusatzstoff, der ein Ether ist, werden die Komponenten Alkohol und Isocyanat aus dem Carbamat-Spaltgas in einem Zeitraum von weniger als 200 Sekunden im Quenchapparat voneinander getrennt. Die Aufgabe des Quenchapparates besteht somit darin, das Carbamat-Spaltgas abzuschrecken und die Komponenten Isocyanat und Alkohol voneinander, insbesondere rektifikativ, zu trennen.

Hierfür wird eine sehr schnelle, praktisch instantane, Abkühlung des Carbamat-Spaltgases durchgeführt.

Die Quench-Verfahrensstufe kann bei einem Betriebsdruck im Bereich von 0,001 bis 20 bar absolut durchgeführt werden.

Bevorzugt wird die Quench-Verfahrensstufe unter Vakuum im Bereich von 1 bis 100 mbar abs. durchgeführt.

Indem erfindungsgemäß als Zusatzstoff in der thermischen Spaltung von Carbamaten ein Ether eingesetzt wird, der als Inhibitor für die Rückreaktion des Isocyanats mit dem Alkohol fungiert, kann mit einer spezifisch geringeren Menge an Zusatzstoff die Bildung von Nebenprodukten, insbesondere die Allophanatbildung, stark zurückgedrängt werden.

Besonders bevorzugt wird der Quench-Verfahrensstufe eine Mischung aus dem Carbamat-Spaltgas und dem Ether zugeführt.

Diese Mischung enthält bevorzugt 0,1 bis 20 Mol-Ether/Mol-Alkohol.

Die Mischung, enthaltend das Carbamat-Spaltgas und den Ether, die der Quenchverfahrensstufe zugeführt wird, hat bevorzugt eine Temperatur im Bereich von 150 bis 500°C und wird vorteilhaft in den Apparat, in dem die Quenchverfahrensstufe durchgeführt wird, entspannt.

Die thermische Spaltung des Carbamats wird bevorzugt in einem Reaktor bei einer Temperatur im Bereich von 150 bis 500°C durchgeführt.

Weiter bevorzugt kann der Quench-Verfahrensstufe ein Gaskühler vorgeschaltet sein, der das Carbamat-Spaltgas bis zur Kondensationsgrenze desselben abkühlt.

Es ist vorteilhaft, die Carbamat-Spaltung in einer Wirbelschicht durchzuführen, die insbesondere im Vakuum betrieben wird.

Die Quench-Verfahrensstufe wird bevorzugt in einem Apparat, der das sehr schnelle Abkühlen (Abschrecken) mit dem flüssigen Ether ermöglicht, insbesondere einem Venturi-Quench, einem Strahlquench, einem Pfeiffenquench, einem Wirbelquench oder einem Rotationsquench durchgeführt, dem eine Quenchkolonne nachgeschaltet ist, worin das gequenchte Carbamat-Spaltgas rektifikativ aufgetrennt wird. Die Gesamtverweilzeit des Carbamat-Spaltgases in der Quench-Verfahrensstufe, d.h. während der schnellen Abkühlung und rektifikativen Auftrennung des abgekühlten Carbamat-Spaltgases, beträgt vorteilhaft weniger als 200 Sekunden.

Bevorzugt wird die Kolonne bei niedrigem Flüssig-Holdup betrieben, wobei der Flüssigkeits-Holdup insbesondere weniger als 6% derselben beträgt. Die Verweilzeit durch das angestaute Flüssigkeitvolumen in der Quenchkolonne wird entscheidend von der Flüssigkeitsbelastung des Quenchmittels beeinflusst. Die Querschnittsbelastung - auch Berieselungsdichte genannt - , d.h. die Flüssigkeitsbelastung bezogen auf den Kolonnenquerschnitt, ist bevorzugt < 20 m³/m²h und besonders bevorzugt < 10 m³/m²h. Die untere Grenze ist durch den Betriebsbereich der verwendeten Kolonneneinbauten gegeben und liegt bei ca. 0,2 m³/m² h.

Der Betriebszustand der Quenchkolonne, charakterisiert durch die Formel ρ_{gas}^{0,5} · u_{gas} [Pa^{0,5}] wird bevorzugt im Bereich von 0,5 bis 2 eingestellt.

In einer weiteren Ausführungsform wird die Quench-Verfahrensstufe in einer Kolonne durchgeführt, und am Sumpf der Kolonne gasförmiger Chlorwasserstoff zusätzlich zum inerten oder inhibierenden Lösungsmittel zur Inhibierung der Rückreaktion des Isocyanats mit dem Alkohol eingespeist.

Die Erfindung wird im Folgenden anhand von Ausführungsbeispielen näher erläutert.

Hierzu wurde die Reaktionsgeschwindigkeit der Urethanisierung von 2,4-Toluylendiisocyanat (2,4-TDI) mit Iso-Butanol in unterschiedlichen Lösungsmitteln untersucht. Die Reaktionskinetik ist bekanntermaßen zweiter Ordnung und die Reaktionsgeschwindigkeit abhängig von der Alkoholkonzentration.

Zunächst wurde die Reaktionsgeschwindigkeit der Urethanisierung von 2,4-TDI in einem Ether untersucht, der im erfindungsgemäßen Verfahren eingesetzt wird:

### Ausführungsbeispiel im Labormaßstab (erfindungsgemäß)

### Urethanisierung von 2,4-TDI in Tetraethylenglykoldimethylether (Tetraglyme):

Ein 250 ml-Vierhalskolben mit Teflonblattrührer, Tropftrichter, Thermometer, Kühler und Heizbad wurde mit Argon überspült. 88,70 g (0,40 Mol) Tetraglyme und 17,40 g (0,10 Mol) 2,4-TDI wurden im Reaktor vorgelegt und auf 40°C erwärmt. Eine Nullprobe wurde genommen und innerhalb von 0,5 min bei 400 U/min 14,79 g (0,20 Mol) Isobutanol zudosiert. Der Isocyanatgehalt wurde durch Probenziehen als Funktion der Zeit verfolgt und die kinetischen Konstanten 2. Ordnung bestimmt.

Die nachfolgende Tabelle 1 zeigt die Abnahme des Isocyanatgehalts als Funktion der Zeit.

**Tabelle 1:**

| T [min] | NCO [Gew-%] | T[°C] |
|---|---|---|
| 0 | 6,9 | 40 |
| 2 | 6,7 | 39 |
| 4 | 6,5 | 42 |
| 30 | 4,9 | 42 |

### Vergleichsbeispiel im Labormaßstab

### Urethanisierung von 2,4-TDI in Chlorbenzol

Zum Vergleich wurde dieselbe Reaktion in derselben Apparatur in gleicher Weise durchgeführt, jedoch abweichend vom obigen Versuch in Chlorbenzol als Lösungsmittel. Hierzu wurden 69,70 g (0,62 Mol) Chlorbenzol mit 17,40 g (0,10 Mol) 2,4-TDI im mit Argon vorgespülten Reaktor vorgelegt und auf 40°C erwärmt. Es wurde eine Nullprobe genommen und innerhalb von 0,5 Minuten bei 400 U/min 14,79 g (0,20 Mol) Isobutanol zudosiert. Der Isocyanatgehalt wurde durch Probenziehen als Funktion der Zeit verfolgt und die kinetischen Konstanten 2. Ordnung bestimmt. Die Abnahme des Isocyanatgehalts in Gewichtsprozent in den ersten Minuten und am Versuchsende als Funktion der Zeit ist in der nachfolgenden Tabelle 2 dargestellt:

**Tabelle 2:**

| T [min] | NCO [Gew%] | T [°C] |
|---|---|---|
| 0 | 8,2 | 40 |
| 2 | 5,6 | 47 |
| 4 | 4,8 | 47 |
| 30 | 3,2 | 41 |

Der Vergleich der in den Tabellen 1 und 2 dargestellten Abnahmen des Isocyanatgehaltes zeigt, dass die Abnahme des Isocyanatgehalts in Gegenwart des Lösungsmittels Chlorbenzol (Vergleichsbeispiel) insbesondere in den ersten vier Minuten signifikant ist und zwar etwa 41% beträgt. Dem gegenüber nimmt der Isocyanatgehalt im erfindungsgemäßen Beispiel, d.h. in Gegenwart von Tetraglyme, lediglich um 4% ab.

Eine Anpassung der Geschwindigkeitskonstanten zweiter Ordnung über die Fehlerquadratregression mit dem Kinetik-Werkzeug Presto ergibt, dass die Geschwindigkeitskonstante für die Reaktion in Gegenwart von Tetraglyme (Ausführungsbeispiel nach der Erfindung) nur ca. 7% der Geschwindigkeitskonstanten für das Vergleichsbeispiel (in Gegenwart von Chlorbenzol) beträgt.

Auch der starke Temperaturunterschied der beiden Reaktionsmischungen beweist die unterschiedliche Reaktivität in Abhängigkeit vom eingesetzten Lösungsmittel.

Im Folgenden wird ein Ausführungsbeispiel für die Durchführung des erfindungsgemäßen Verfahrens in einer Quenchkolonne beschrieben.

### Ausführungsbeispiel im technischen Maßstab

In einer Spaltapparatur wird 2,4-Toluylen-bis(O-isobutylcarbamat) kontinuierlich gespalten und anschließend gequencht. Sie Spaltung erfolgt bei 400°C mit einer Wirbelschicht. Das so gewonnene Spaltgas wird gekühlt, bevor es in den Quench eingeleitet wird.

Eine Kolonne aus austenitischem Stahl mit 6 theoretischen Trennstufen und einem Durchmesser von 60 mm ist mit einer Blechpackung mit niedrigem Flüssig-Holdup ausgestattet, um die Verweilzeit gering zu halten. Das Produkt aus ρ_{gas}^{0,5}· u_{gas}.bewegt sich in der Kolonne im Bereich von 0,5 bis 1,7 Pa^{0,5}, wobei u_{gas} = Leerrohrgeschwindigkeit des aufsteigenden Gases, ρ_{gas} = Dichte des aufsteigenden Gases. Der Quench wird bei 30 mbar betrieben. Die Berieselungsichte beträgt ca. 7 m³/m²h.

53,9 mol/h eines Spaltgases enthaltend 58mol% Butanol und 28,7mol% 2,4-TDI (Rest N₂ als Wirbelgas) werden gasförmig mit 280°C auf die 2. Stufe des Carbamatspaltgas-Quenchs gegeben. Am Kolonnenkopf werden 81,0 mol/h Tetraglyme flüssig bei 20°C auf den Verteiler gegeben. Am Kolonnenkopf wird ein gasförmiger Strom von 38,3 mol/h abgezogen. Am Kopf finden sich 31,1 mol/h Isobutanol. Der Anteil an Tetraglyme im Kopf beträgt 0,05 mol/h.

Am Kolonnensumpf werden 96,5 mol/h bei 155°C abgezogen. Dort finden sich 15,4 mol/h 2,4-TDI.

Der Anteil an Allophanaten und Isocyanuraten am Sumpf der Kolonne beträgt weniger als 2 mol% bezogen auf 2,4-TDI.

## Patentansprüche

1. Verfahren zur Herstellung von Isocyanaten durch thermische Spaltung von Carbamaten, wobei ein Carbamat-Spaltgas, enthaltend das entsprechende Isocyanat und den entsprechenden Alkohol, erhalten wird, **dadurch gekennzeichnet, dass** das Carbamat-Spaltgas in Gegenwart eines Ethers, der als Inhibitor für die Rückreaktion des Isocyanats mit dem Alkohol fungiert, gequencht wird.

2. Verfahren nach Anspruch 1, **dadurch gekennzeichnet, dass** als Ether ein Polyalkylenglykoldialkylether und/oder ein Polyalkylenglykoldiarylether eingesetzt wird.

3. Verfahren nach Anspruch 2, **dadurch gekennzeichnet, dass** als Polyalkylenglykoldialkylether ein Polyethylenglykoldialkylether eingesetzt wird.

4. Verfahren nach Anspruch 3, **dadurch gekennzeichnet, dass** als Polyethylenglykoldialkylether eine oder mehrere der nachfolgend aufgeführten Substanzen eingesetzt wird: Diethylenglykoldimethylether, Triethylenglykoldimethylether und Tetraethylenglykoldimethylether.

5. Verfahren nach Anspruch 4, **dadurch gekennzeichnet, dass** als Ether Tetraethylenglykoldimethylether eingesetzt wird.

6. Verfahren nach Anspruch 2, **dadurch gekennzeichnet, dass** als Ether ein Polypropylenglykoldialkylether und/oder ein Polypropylenglykoldiarylether eingesetzt wird.

7. Verfahren nach Anspruch 6, **dadurch gekennzeichnet, dass** als Polypropylenglykoldialkylether eine oder mehrere Substanzen, ausgewählt aus der nachfolgenden Aufzählung eingesetzt werden: Dipropylenglykoldimethylether, Tripropylenglykoldimethylether oder Tetrapropylenglykoldimethylether.

8. Verfahren nach Anspruch 1, **dadurch gekennzeichnet, dass** der Ether eine oder mehrere Substanzen, ausgewählt aus der nachfolgenden Aufzählung ist: Oxolan, Dioxolan, Trioxolan, Diarylether, Alkylarylether, Diphenylether, Ditoluylether und Dibenzylether.

9. Verfahren nach einem der Ansprüche 1 bis 5, **dadurch gekennzeichnet, dass** es sich bei den Carbamaten um N,N'-Toluylen-bis(O-alkylurethan), N,N'-Toluylen-bis(O-alkoxalkylyurethan), N,N'-Toluylenbis(O-arylurethan) N,N'-1,5-Naphthalin-bis(O-dialkylurethan), N,N'-1,5-Naphthalin-bis(O-alkoxylalkylurethan), N,N'-1,5-Naphthalin-bis(O-arylurethan), Hexamethylen-bis(O-alkylurethan), Hexamethylen-bis(O-alkoxyalkylurethan), Isophoron-bis(O-alkylurethan), Isophoron-bis(O-alkoxyalkylurethan), 4,4'-Diphenylmethandi-O-alkylurethan oder 4,4'-Diphenylmethan,di-O-alkoxyalkylurethan handelt.

10. Verfahren nach einem der Ansprüche 1 bis 9, **dadurch gekennzeichnet, dass** der Quench-Verfahrensstufe eine Mischung aus dem Carbamat-Spaltgas und dem Ether zugeführt wird.

11. Verfahren nach Anspruch 7, **dadurch gekennzeichnet, dass** die Mischung aus dem Carbamat-Spaltgas und dem Ether 0,1 bis 20 Mol Ether pro Mol Alkohol enthält.

12. Verfahren nach Anspruch 10 oder 11, **dadurch gekennzeichnet, dass** die Mischung aus dem Carbamat-Spaltgas und dem Ether eine Temperatur von 150 bis 500°C hat und bei der Zuführung in die Quench-Verfahrensstufe entspannt wird.

13. Verfahren nach einem der Ansprüche 1 bis 12, **dadurch gekennzeichnet, dass** die thermische Spaltung des Carbamats in einem Reaktor bei einer Temperatur im Bereich von 150 bis 500°C durchgeführt wird.

14. Verfahren nach einem der Ansprüche 1 bis 13, **dadurch gekennzeichnet, dass** der Quench-Verfahrensstufe ein Gaskühler vorgeschaltet ist, worin das Carbamat-Spaltgas bis zur Kondensationsgrenze desselben abgekühlt wird.

15. Verfahren nach einem der Ansprüche 1 bis 14, **dadurch gekennzeichnet, dass** die Gesamtverweilzeit in der Quench-Verfahrensstufe kleiner als 200 Sekunden ist.

## Claims

1. A process for preparing isocyanates by thermally cleaving carbamates to obtain a carbamate cleavage gas comprising the corresponding isocyanate and the corresponding alcohol, which comprises quenching the carbamate cleavage gas in the presence of an ether which functions as an inhibitor for the reverse reaction of the isocyanate with the alcohol.

2. The process according to claim 1, wherein the ether used is a polyalkylene glycol dialkyl ether and/or a polyalkylene glycol diaryl ether.

3. The process according to claim 2, wherein the polyalkylene glycol dialkyl ether used is a polyethylene glycol dialkyl ether.

4. The process according to claim 3, wherein the polyethylene glycol dialkyl ether used is one or more of the following substances: diethylene glycol dimethyl ether, triethylene glycol dimethyl ether and tetraethylene glycol dimethyl ether.

5. The process according to claim 4, wherein the ether used is tetraethylene glycol dimethyl ether.

6. The process according to claim 2, wherein the ether used is a polypropylene glycol dialkyl ether and/or a polypropylene glycol diaryl ether.

7. The process according to claim 6, wherein the polypropylene glycol dialkyl ether used is one or more substances selected from the following list: dipropylene glycol dimethyl ether, tripropylene glycol dimethyl ether and tetrapropylene glycol dimethyl ether.

8. The process according to claim 1, wherein the ether is one or more substances selected from the following list: oxolane, dioxolane, trioxolane, diaryl ether, alkyl aryl ether, diphenyl ether, ditolyl ether and dibenzyl ether.

9. The process according to any of claims 1 to 5, wherein the carbamates are N,N'-tolylenebis(O-alkylurethane), N,N'-tolylenebis(O-alkoxalkylurethane), N,N'-tolylenebis(O-arylurethane), N,N'-1,5-naphthalenebis(O-dialkylurethane), N,N'-1,5-naphthalenebis(O-alkoxylalkylurethane), N,N'-1,5-naphthalenebis(O-arylurethane), hexamethylenebis(O-alkylurethane), hexamethylenebis(O-alkoxyalkylurethane), isophoronebis(O-alkylurethane), isophoronebis(O-alkoxyalkylurethane), 4,4'-diphenylmethanedi-O-alkylurethane or 4,4'-diphenylmethanedi-O-alkoxyalkylurethane.

10. The process according to any of claims 1 to 9, wherein a mixture of the carbamate cleavage gas and the ether is supplied to the quench process stage.

11. The process according to claim 7, wherein the mixture of the carbamate cleavage gas and the ether comprises 0.1 to 20 mol of ether per mole of alcohol.

12. The process according to claim 10 or 11, wherein the mixture of the carbamate cleavage gas and the ether has a temperature of 150 to 500°C and is decompressed when supplied to the quench process stage.

13. The process according to any of claims 1 to 12, wherein the thermal cleavage of the carbamate is performed in a reactor at a temperature in the range from 150 to 500 °C.

14. The process according to any of claims 1 to 13, wherein the quench process stage is preceded upstream by a gas cooler in which the carbamate cleavage gas is cooled down to the condensation limit thereof.

15. The process according to any of claims 1 to 14, wherein the total residence time in the quench process stage is less than 200 seconds.

## Revendications

1. Procédé pour la préparation d'isocyanates par dissociation thermique de carbamates, un gaz de dissociation du carbamate, contenant l'isocyanate correspondant et l'alcool correspondant, étant obtenu, **caractérisé en ce que** le gaz de dissociation du carbamate est refroidi brusquement en présence d'un éther qui fonctionne comme inhibiteur pour la réaction en retour de l'isocyanate avec l'alcool.

2. Procédé selon la revendication 1, **caractérisé en ce qu'**on utilise, comme éther, un polyalkylèneglycoldialkyléther et/ou un polyalkylèneglycoldiaryléther.

3. Procédé selon la revendication 2, **caractérisé en ce qu'**on utilise, comme polyalkylèneglycoldialkyléther, un polyéthylèneglycoldialkyléther.

4. Procédé selon la revendication 3, **caractérisé en ce qu'**on utilise, comme polyéthylèneglycoldialkyléther, une ou plusieurs des substances indiquées ci-après : diéthylèneglycoldiméthyléther, triéthylèneglycoldiméthyléther et tétraéthylèneglycoldiméthyléther.

5. Procédé selon la revendication 4, **caractérisé en ce qu'**on utilise, comme éther, du tétraéthylèneglycoldiméthyléther.

6. Procédé selon la revendication 2, **caractérisé en ce qu'**on utilise, comme éther, un polypropylèneglycoldialkyléther et/ou un polypropylèneglycoldiaryléther.

7. Procédé selon la revendication 6, **caractérisé en ce qu'**on utilise, comme polypropylèneglycoldialkyléther, une ou plusieurs substances, choisies parmi l'énumération suivante : dipropylèneglycoldiméthyléther, tripropylèneglycoldiméthyléther ou tétrapropylèneglycoldiméthyléther.

8. Procédé selon la revendication 1, **caractérisé en ce que** l'éther est une ou plusieurs substances, choisies parmi l'énumération suivante : oxolane, dioxolane, trioxolane, diaryléther, alkylaryléther, diphényléther, ditoluyléther et dibenzyléther.

9. Procédé selon l'une quelconque des revendications 1 à 5, **caractérisé en ce qu'**il s'agit, pour les carbamates, de N,N'-toluylène-bis(O-alkyluréthane), de N,N'-toluylène-bis(O-alcoxyalkyluréthane), de N,N'-toluylène-bis(O-aryluréthane), de N,N'-1,5-naphtalène-bis(O-dialkyluréthane), de N,N'-1,5-naphtalène-bis(O-alcoxyalkyluréthane), de N,N'-1,5-naphtalène-bis(O-aryluréthane), d'hexaméthylène-bis(O-alkyluréthane), d'hexaméthylène-bis(O-alcoxyalkyluréthane), d'isophorone-bis(O-alkyluréthane), d'isophorone-bis(O-alcoxyalkyluréthane), de 4,4'-diphénylméthanedi-O-alkyluréthane ou de 4,4'-diphénylméthanedi-O-alcoxyalkyluréthane.

10. Procédé selon l'une quelconque des revendications 1 à 9, **caractérisé en ce que** l'étape de procédé de refroidissement brusque est alimentée en un mélange constitué par le gaz de dissociation du carbamate et l'éther.

11. Procédé selon la revendication 7, **caractérisé en ce que** le mélange du gaz de dissociation du carbamate et de l'éther contient 0,1 à 20 moles d'éther par mole d'alcool.

12. Procédé selon la revendication 10 ou 11, **caractérisé en ce que** le mélange du gaz de dissociation du carbamate et de l'éther présente une température de 150 à 500°C et est détendu lors de l'alimentation dans l'étape de procédé de refroidissement brusque.

13. Procédé selon l'une quelconque des revendications 1 à 12, **caractérisé en ce que** la dissociation thermique du carbamate est réalisée dans un réacteur à une température dans la plage de 150 à 500°C.

14. Procédé selon l'une quelconque des revendications 1 à 13, **caractérisé en ce que** l'étape de procédé de refroidissement brusque est précédée d'un refroidisseur de gaz, dans lequel le gaz de dissociation du carbamate est refroidi jusqu'à la limite de condensation de celui-ci.

15. Procédé selon l'une quelconque des revendications 1 à 14, **caractérisé en ce que** la durée de séjour totale dans l'étape de procédé de refroidissement brusque est inférieure à 200 secondes.
